**Europäisches Patentamt**

**European Patent Office**

**Office européen des brevets**

(19)

(11) Veröffentlichungsnummer : **0 366 603 B1**

(12)

# EUROPÄISCHE PATENTSCHRIFT

(45) Veröffentlichungstag der Patentschrift :
**12.08.92 Patentblatt 92/33**

(51) Int. Cl.⁵ : **A61F 2/36,** A61F 2/30

(21) Anmeldenummer : **89810746.1**

(22) Anmeldetag : **02.10.89**

(54) **Blattartiger Schaft aus Metall für eine Femurkopfprothese.**

(30) Priorität : **26.10.88 CH 3985/88**

(43) Veröffentlichungstag der Anmeldung :
**02.05.90 Patentblatt 90/18**

(45) Bekanntmachung des Hinweises auf die
Patenterteilung :
**12.08.92 Patentblatt 92/33**

(84) Benannte Vertragsstaaten :
**AT DE FR GB IT**

(56) Entgegenhaltungen :
**EP-A- 0 149 527**
**EP-A- 0 196 258**

(56) Entgegenhaltungen :
**EP-A- 0 217 034**
**EP-A- 0 266 081**
**EP-A- 0 273 871**
**FR-A- 2 610 824**

(73) Patentinhaber : **GEBRÜDER SULZER**
**AKTIENGESELLSCHAFT**
**Zürcherstrasse 9**
**CH-8401 Winterthur (CH)**

(72) Erfinder : **Frey, Otto, Dr.**
**Wallrütistrasse 56**
**CH-8400 Winterthur (CH)**
Erfinder : **Koch, Rudolf**
**Oberdorfstrasse 229**
**CH-8267 Berlingen (CH)**

**Beschreibung**

Die Erfindung betrifft einen blattartigen Schaft aus Metall für eine Femurkopfprothese mit einer Oberflächenstruktur im proximalen Bereich zum An- und/oder Einwachsen von Knochengewebe, wobei mit Führungen versehene Ausnehmungen vorhanden sind, in die die Aussenform des Schaftes in diesen Bereich gestaltende, mit einem ein- oder mehrlagigen Metallgitter, insbesondere mit einem Drahtnetz, belegte, separate Kunststoff-Formstücke einschiebbar sind.

Ein Verankerungsschaft der vorstehend genannten Art ist beispielsweise bekannt aus der EP-A-217 034; wird ein solcher Schaft zementfrei implantiert, so dass eine direkte Grenzfläche zwischen Knochengewebe und der Metallgitterstruktur vorhanden ist, in die Knochengewebe einwächst, so treten in dieser Grenzfläche bei Biegebeanspruchungen des Schaftes Scherkräfte auf, die das durch diese Grenzfläche wachsende Gewebe belasten.

Aufgabe der Erfindung ist es, für einen Schaft der eingangs genannten Art eine Konstruktion zu schaffen, bei der in der Grenzfläche zum angrenzenden Knochengewebe keine durchwachsendes Gewebe belastenden Scherkräfte auftreten. Mit der Erfindung wird diese Aufgabe dadurch gelöst, dass die als Führungen ausgebildeten, proximalen und distalen Begrenzungen einer Ausnehmung Sektoren zweier konzentrischer Kreisbogen sind, deren Zentrum distal von den Begrenzungen gelegen ist.

Exzentrisch zur Schaftachse auf den Gelenkkopf wirkende Kräfte, die den Schaft auf Biegung beanspruchen, verursachen bei der neuen Konstruktion Relativbewegungen zwischen den Kunststoff-Formstücken und dem "Restschaft". Diese Relativbewegungen sind in den kreisförmigen Führungen geführte "Drehungen" der Formstücke gegenüber dem Schaft. Dabei verschieben sich die Rückseite des Formstücke und die Fläche einer Ausnehmung des Schaftes relativ zueinander, während Relativbewegungen in der Grenzfläche Knochen/Oberflächenstruktur der Formstücke zumindest weitgehend aufgehoben werden, so dass das Gewebe schädigende Scherbelastungen dort nicht auftreten.

Für eine Entlastung der Grenzfläche zwischen der Oberflächenstruktur und dem Gewebe hat es sich als besonders zweckmässig erwiesen, wenn die Führungen von lateral nach medial über die ganze Blattseite verlaufen. Gegen unbeabsichtiges Herausrutschen der Formstücke aus den Führungen kann auf der Basis der Formstücke ein zapfenartiger Ansatz vorgesehen sein, der mit Spiel in eine Vertiefung der Ausnehmung eingreift.

Um den neuen Schaft ohne Aenderungen für verschieden grosse operative Hohlräume im Femur und/oder beidseitig für Hüftgelenke einsetzen zu können, ist es zweckmässig, wenn Formstücke unterschiedlicher Dikken vorhanden sind, wobei die Dicken jeder einzelnen "Grösse" gegebenenfalls von distal nach proximal zunehmen können.

Im folgenden wird die Erfindung anhand eines Ausführungsbeispiels im Zusammenhang mit der Zeichnung näher erläutert.

Fig. 1 zeigt eine Ansicht in Richtung von frontal/dorsal des in einen Femurknochen eingesetzten neuen Schaftes;

Fig. 2 ist eine Ansicht von Fig. 1 von links;

Fig. 3 schliesslich gibt, vergrössert, den Schnitt III-III von Fig. 1 wieder.

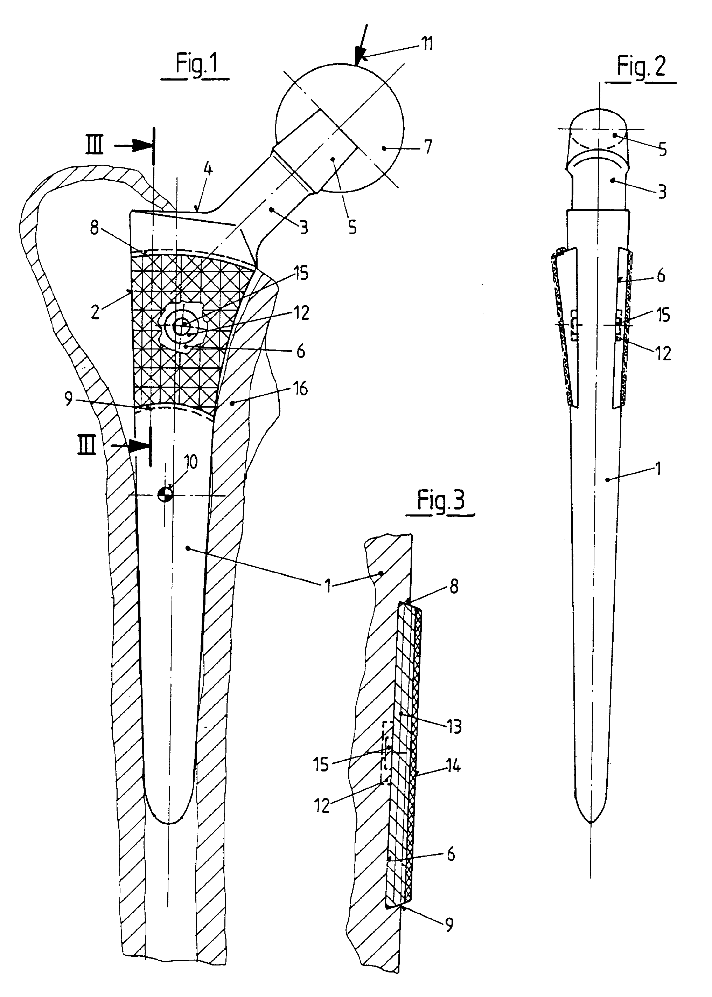

Der in einen schematisch angedeuteten Femur 16 eingesetzte Geradschaft 1 (Fig. 1) verjüngt sich nach distal; seine laterale Schmalseite setzt sich an seinem proximalen Ende über eine horizontale Schulter 4 in den Prothesenhals 3 fort, der seinerseits in einem konischen Zapfen 5 zur Aufnahme eines nur angedeuteten Gelenkkopfes 7 endet.

Im proximalen Bereich der beiden Blattseiten weist der Schaft 1 Ausnehmungen 6 auf. Diese sind distal und proximal durch als konzentrische Kreisbögen ausgebildete Führungen 8 und 9 begrenzt, deren Mittelpunkt 10 distal der Führungen 8 und 9, etwa im "Drehzentrum" der Biegungen des Schaftes 1 liegt, die durch die auf den Gelenkkopf 7 wirkenden Belastungen (Pfeil 11) verursacht werden. Die Führungen 8 und 9 bilden zusammen eine schwalbenschwanzförmige Führungsnut (Fig. 3). Im Boden jeder Ausnehmung 6 ist eine kreisförmige Vertiefung 12 vorgesehen.

Als Füllung für die Ausnehmungen 6 dienen Formstücke 13, die medial und lateral an die Schaftform angepasst sind und in ihrer Dicke von distal nach proximal zunehmen (Fig. 2). Sie sind aus Kunststoff gefertigt und auf ihren Aussenflächen mit einer Oberflächenstruktur versehen, die beispielsweise aus einem ein- oder mehrlagigen Metallgitter oder Drahtnetz 14 besteht. Für einen Eingriff in die von den Führungen 8 und 9 gebildete Führungsnut erweitern sich die Kunststoff-Formstücke zu einem Ende konisch. Gegen ein unbeabsichtigtes Ausschieben aus der Führungsnut sind die Formstücke 13 am Boden mit einem zapfenartigen Vorsprung 15 versehen, der mit Spiel in die Vertiefung 12 eingreift.

Wie Fig. 2 erkennen lässt, können die Formstücke 13 unterschiedliche Dicke haben, so dass in eine Blattseite ein dickes und die andere ein dünnes Formstück eingesetzt werden kann; dies ermöglicht den gleichen Schaft für unterschiedlich grosse Operationshohlräume und/oder für linke und rechte Hüften zu verwenden.

**Patentansprüche**

1. Blattartiger Schaft aus Metall für eine Femurkopfprothese mit einer Oberflächenstruktur im proximalen Bereich zum An- und/oder Einwachsen von Knochengewebe, wobei mit Führungen (8, 9) versehene Ausnehmungen (6) vorhanden sind, in die die Aussenform des Schaftes (1) in diesem Bereich gestaltende, mit einem ein- oder mehrlagigen Metallgitter, insbesondere mit einem Drahtnetz (14), belegte, separate Kunststoff-Formstücke (13) einschiebbar sind, dadurch gekennzeichnet, dass die als Führungen (8, 9) ausgebildeten, proximalen und distalen Begrenzungen einer Ausnehmung (6) Sektoren zweier konzentrischer Kreisbögen sind, deren Zentrum (10) distal von den Begrenzungen (8, 9) gelegen ist.

2. Schaft nach Anspruch 1, dadurch gekennzeichnet, dass die Führungen (8,9) von lateral nach medial über die ganze Blattseite verlaufen.

3. Schaft nach Anspruch 1 oder 2, dadurch gekennzeichnet, dass auf der Basis der Formstücke (13) ein zapfenartiger Ansatz (15) vorgesehen ist, der mit Spiel in eine Vertiefung (12) der Ausnehmung (6) eingreift.

4. Schaft nach einem der Ansprüche 1 bis 3, dadurch gekennzeichnet, dass die Formstücke (13) unterschiedliche Dicken haben.

5. Schaft nach einem der Ansprüche 1 bis 4, dadurch gekennzeichnet, dass die Dicke eines Formstückes (13) von distal nach proximal zunimmt.

**Claims**

1. A blade-like metal stem for a femoral head prosthesis having in the proximal zone a surface structure promoting the ongrowth and/or invasion of bone tissue, recessed parts (6) being present which have guides (8, 9) into which separate plastics mouldings (13) which make up the external shape of the stem (1) in this zone and which are covered with a single-layer or multilayer metal mesh, more particularly a wire netting (14), are introducible, characterised in that the proximal and distal boundaries, contrived as guides (8, 9), of a recessed part (6) are sectors of two concentric circle arcs whose centre (10) is disposed distally in respect of the boundaries (8, 9).

2. A stem according to claim 1, characterised in that the guides (8, 9) extend laterally to medially over the whole side of the blade.

3. A stem according to claim 1 or 2, characterised in that a pin-like projection (15) is disposed on the base of the mouldings (13) and engages with clearance in a deepening (12) in the recessed part (6).

4. A stem according to any of claims 1 to 3, characterised in that the mouldings (13) are of different thicknesses.

5. A stem according to any of claims 1 to 4, characterised in that the thickness of a moulding (13) increases distally to proximally.

**Revendications**

1. Tige métallique en lame pour une prothèse de tête de fémur présentant une surface structurée dans la région proximale pour la croissance couvrante et/ou pénétrante du tissu osseux, des évidements (6), pourvus de guides (8, 9), étant prévus et dans lesquels peuvent être insérées des pièces moulées en matière plastique (13) séparées, conférant la forme extérieure de la tige (1) dans cette région, garnies d'une grille métallique en une ou plusieurs couches, en particulier d'un filet métallique (14), caractérisée en ce que les délimitations proximales et distales, configurées en guides (8, 9), d'un évidement (6) sont des secteurs de deux arcs de cercles concentriques dont le centre (10) est situé distalement par rapport aux délimitations (8, 9).

2. Tige selon la revendication 1, caractérisée en ce que les guides (8, 9) s'étendent de la région latérale à la région médiale, sur toute la face de la lame.

3. Tige selon la revendication 1 ou 2, caractérisée en ce qu'il est prévu, sur la base des pièces moulées (13), un appendice (15) en forme de tenon, qui s'engage avec un certain jeu dans un creux (12) de l'évidement (6).

4. Tige selon l'une des revendications 1 à 3, caractérisée en ce que les pièces moulées (13) ont des épaisseurs différentes.

5. Tige selon l'une des revendications 1 à 4, caractérisée en ce que l'épaisseur d'une pièce moulée (13) augmente de la région distale à la région proximale.

Fig.1

Fig.2

Fig.3